# EUROPEAN PATENT APPLICATION

(11) **EP 4 523 713 A1**
(43) Date of publication of application: **19.03.2025**
(21) Application number: 22941063.4
(22) Date of filing: 10.05.2022
(51) Int. Cl.: A61L 27/20, A61L 27/24, A61L 27/50, A61L 27/52, A61L 27/54, A61L 27/56

(54) **INJECTABLE ANTI-HEART FAILURE HYDROGEL HAVING MYOCARDIAL TISSUE REPAIR FUNCTIONALITY, METHOD FOR PREPARING SAME, AND USE THEREOF**

(30) Priority: 07.05.2022 CN 202210495091
(71) Applicant: Sichuan University, Chengdu, Sichuan 610065 (CN)
(72) Inventor: WANG, Yunbing, Chengdu, Sichuan 610065 (CN); YANG, Li, Chengdu, Sichuan 610065 (CN); SHAO, Nan, Chengdu, Sichuan 610065 (CN); YANG, Xia, Chengdu, Sichuan 610065 (CN); ZHANG, Xingdong, Chengdu, Sichuan 610065 (CN)
(74) Representative: Zaboliene, Reda
(86) International application number: PCT/CN2022/091946
(87) International publication number: WO 2023/216099

(57) **Abstract**

An injectable hydrogel for heart failure treatment with myocardial tissue repair function and its preparation method and use are disclosed. The hydrogel is prepared by chelating alginic acid and multivalent cations. The hydrogel is loaded with an active substance for repairing cardiac injury. The active substance is recombinant humanized collagen type III. The hydrogel is administrated into the left ventricle free wall by transendocardial injection. Implanting the hydrogel in the myocardium can increase the thickness of the left ventricular wall, adjust the stress of the ventricular wall, affect the morphology of the left ventricle, and prevent or reverse left ventricular enlargement. Further, the hydrogel is loaded with a new recombinant humanized collagen material that has the function of repairing cardiac tissue injury to further improve cardiac function and promote cardiac repair.

## Description

### TECHNICAL FIELD

The present disclosure belongs to the technical field of biomedical materials, and in particular relates to an injectable anti-heart failure hydrogel with myocardial tissue repair function, preparation method and use thereof.

### DESCRIPTION OF THE PRIOR ART

Heart failure (HF) refers to systolic/diastolic dysfunction of the heart, insufficient blood supply of the heart, and inability to provide the needed blood flow, which is a clinical manifestation of end-stage of almost all cardiovascular diseases such as coronary heart disease, hypertension, structural heart disease, and myocardial infarction. The treatment of heart failure remains an unsolved huge challenge in the cardiovascular field.

The existing treatment options for heart failure are very limited, and can be generally divided into two categories: drug treatment and non-drug treatment. Drug treatment can effectively improve myocardial contractility and reduce cardiac load, but cannot curb the continuous attenuation of left ventricular function. Long-term administration can cause drug side effects and has limited efficacy. Traditional non-drug treatments use left ventricular assist devices, implantable cardioverter defibrillators, heart transplants, etc., which can overcome the shortcomings of drug treatment to a certain extent, prevent arrhythmia and increase blood flow, thereby alleviating symptoms, but cannot fundamentally cure heart failure. Especially the heart transplants are rarely used for surgery due to problems such as scarcity of donors and immune rejection. With the development of tissue engineering and regenerative medicine, implantable materials have been effectively used for cardiac repair. Biomaterials such as hydrogels, collagen, extracellular matrix, and patches have been widely used in heart failure treatment. Further, combining biomaterials with different active substances can effectively improve the treatment efficiency of heart failure, which has great development prospects and application prospects in the field of biomedicine.

### SUMMARY OF THE DISCLOSURE

Implantable materials have been effectively used for cardiac repair. The present disclosure provides an injectable anti-heart failure hydrogel with myocardial tissue repair function and a preparation method thereof. The hydrogel is administrated to the left ventricular free wall by transendocardial injection. Implanting the hydrogel in the myocardium can increase the thickness of the left ventricular wall, adjust the stress of the ventricular wall, affect the morphology of the left ventricle, and prevent or reverse left ventricular enlargement. Further, the hydrogel is loaded with a new recombinant humanized collagen material that has the function of repairing cardiac tissue injury to further improve cardiac function and promote cardiac repair.

The hydrogel is prepared by chelating alginic acid and multivalent cations, and the hydrogel is loaded with an active substance for repairing cardiac injury.

Alginic acid is a natural polysaccharide with advantages of being cheap, easy access, renewable, and biocompatible. It is a copolymer composed of α-L-mannuronic acid and β-D-guluronic acid connected by 1, 4-glycosidic bonds and contains many carboxylic acid groups, and can form a hydrogel after being chelated by multivalent cations (such as divalent cations).

The hydrogel formed by alginic acid and multivalent cations has good biocompatibility, less immune response of the body, good injectability and a certain degree of biological inertness, and can be effectively loaded with biologically active substances. Based on the different loaded active substances, at least one of repairing cardiac injury, promoting cardiac angiogenesis, and improving cardiac function can be achieved.

The hydrogel loaded with an active substance has good biocompatibility under physiological conditions and can be administrated to the cardiac injury site in various ways, including but not limited to: being filled in a syringe or delivery system and directly injected into the ventricular wall.

After the hydrogel reaches the cardiac injury site through injection, it not only provides effective mechanical support, but the loaded active substance also has a significant function of promoting cell growth, effectively promoting cardiac angiogenesis and repair.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

Appropriate water content ensures that the hydrogel has superior rheological properties and injectability, making it easy to be filled in the syringe or delivery system and delivered to the cardiac injury site for treatment.

Optionally, the hydrogel is prepared by mixing an alginate solution with an aqueous solution containing calcium ions, and the volume ratio between the alginate solution and the aqueous solution containing calcium ions is in the range of 2:1 to 6:1.

Optionally, the concentration of alginate in the alginate solution is in the range of 0.5-5 wt%.

Optionally, the concentration of alginate in the alginate solution is in the range of 1-3 wt%.

Optionally, the concentration of calcium ions in the aqueous solution containing calcium ions is in the range of 0.5-2 wt%.

Optionally, the concentration of calcium ions in the aqueous solution containing calcium ions is in the range of 1-2 wt%.

Optionally, the alginate is sodium alginate, and the solute of the aqueous solution containing calcium ions is at least one of calcium lactate, calcium carbonate, calcium gluconate, calcium citrate, and calcium chloride.

Optionally, the load concentration of the active substance in the hydrogel is in the range of 1 to 10 g/L.

Optionally, the load concentration of the active substance in the hydrogel is in the range of 1-5 g/L.

Optionally, the active substance is: a protein containing an amino acid sequence fragment that can bind to cellular integrins.

The active substance has at least one of the following effects:
a) Promoting repair of injured heart;
b) Adjuvant therapy for heart failure;
c) Promoting cell proliferation in injured heart area;
b) Promoting angiogenesis;
e) Remodeling of injured heart.

Optionally, the active substance is recombinant humanized collagen type III.

The recombinant humanized collagen type III is prepared through screening and biosynthetic means. It has a collagen functional region with the same amino acid sequence and structure as human collagen type III, and can promote cell growth. Compared with animal collagen, it can reduce the immunogenicity of animal-derived tissues and repair cardiac injury.

Optionally, the load concentration of recombinant humanized collagen type III in the hydrogel is in the range of 1-10 g/L.

Optionally, the recombinant humanized collagen type III contains an amino acid sequence fragment that can bind to cellular integrins.

Recombinant humanized collagen type III refers to a part or the whole of the amino acid sequence fragment encoded by a specific type of gene of human collagen type III prepared by DNA recombinant technology, or a combination containing functional fragments of human collagen.

Optionally, the amino acid fragment sequence of the recombinant humanized collagen type III is GERGAPGFRGPAGPNGIPGEKGPAGERGAP.

The recombinant humanized collagen type III has no significant cytotoxicity and has low immune rejection in the human body. Because its structure has hydrophilic functional groups such as carboxyl, amino and guanidine groups, and its structure has concentrated positive and negative charges, it has a high water-solubility and cell adhesion activity, and has high endothelial cell affinity. It is a customized collagen that can be used to modify cardiovascular materials.

The recombinant humanized collagen type III has a significant cell growth-promoting effect. Compared with animal collagen, it can not only reduce the immunogenicity of animal-derived tissues, but also repair cardiac injury.

Recombinant humanized collagen type III is an important component of the human extracellular matrix and is widely distributed in the vascular system and internal organs. Research has found that collagen type III is a key component for wound healing and tissue regeneration. It is widely used in tissue engineering research and tissue regeneration field. Animal-derived collagen type III has limitations such as immunogenicity, differences among different batches, and poor water-solubility. In contrast, recombinant humanized collagen type III has the same amino acid sequence as collagen derived from human tissue and is safer. Therefore, recombinant humanized collagen type III is preferred in tissue engineering applications. However, recombinant humanized collagen type III has low mechanical properties and is likely to be enzymatically degraded in the human body, which greatly weakens the efficacy of recombinant humanized collagen type III in tissue repair and regeneration.

Optionally, the storage modulus of the hydrogel is in the range of 100-7000 Pa.

Optionally, the hydrogel has a three-dimensional mesh structure, and the mesh aperture is in the range of 30-100 µm.

The multivalent cations are calcium ions. Calcium alginate hydrogel, as a natural, non-toxic and biocompatible material, will not be hydrolyzed by enzymes in the human body. Its unique three-dimensional mesh microstructure can deliver nutrients to the tissue, and such material also has good mechanical properties and can support cell proliferation and migration. However, the repair and regeneration functions of calcium alginate hydrogels for myocardial cells still need to be improved.

Optionally, the load content of recombinant humanized collagen type **III** in the hydrogel is in the range of 0.1-1 wt%.

Optionally, the load concentration of recombinant humanized collagen type **III** in the hydrogel is in the range of 1 to 10 g/L.

The present disclosure further provides a preparation method of the injectable anti-heart failure hydrogel with myocardial tissue repair function, including:

mixing an alginate solution with a multivalent cation solution, wherein the alginate solution and/or the multivalent cation solution contains an active substance, so as to obtain the injectable anti-heart failure hydrogel with myocardial tissue repair function.

The preparation is performed at room temperature, that is, no additional heating or cooling is required. Room temperature is usually between -10 °C and 40 °C. Dissolving and mixing must be performed completely.

The calcium alginate hydrogel for heart failure treatment according to the present disclosure is a hydrogel with a high water content formed by uniformly mixing the alginate solution loaded with a biologically active substance with the aqueous solution containing calcium ions in a sterile environment, which is then filled into a syringe or delivery system and injected directly into the ventricular wall for treatment.

The hydrogel is prepared in a sterile environment, and the alginic acid is chelated by the multivalent cations to form the hydrogel.

The active substance can be premixed in an alginic acid solution or in the multivalent cation solution, and then the alginic acid solution and the multivalent cation solution are mixed.

Optionally, the alginic acid solution containing the active substance and the multivalent cation solution are mixed to obtain the injectable anti-heart failure hydrogel with myocardial tissue repair function.

Optionally, the alginate is sodium alginate, and the solute of the aqueous solution containing calcium ions is at least one of calcium lactate, calcium carbonate, calcium gluconate, calcium citrate, and calcium chloride.

Optionally, the active substance is recombinant humanized collagen type III**.**

Optionally, the solvent of the alginate solution and the solvent of the multivalent cation solution are the same, and the solvent is water or PBS buffer.

Optionally, the active substance is dissolved in the alginate solution and then mixed with the aqueous solution containing calcium ions.

Optionally, the alginate solution is a PBS solution of sodium alginate. In the PBS solution of sodium alginate, the concentration of sodium alginate is in the range of 0.5-5 wt%.

The molecular formula of sodium alginate is (C₆H₇NaO₆)ₓ. The sterile, pyrogen-free sodium alginate is dissolved in PBS under sterile conditions at room temperature and sufficiently solubilized to obtain the PBS solution of sodium alginate. The dissolution time is determined depending on the actual dosage, such as 12 hours or 24 hours.

Optionally, the multivalent cation solution is a PBS solution of calcium ions, and the concentration of calcium ions in the PBS solution of calcium ions is in the range of 00.5-2% wt%.

Under sterile conditions at room temperature, the sterile, pyrogen-free calcium salt is dissolved in PBS and sufficiently solubilized to obtain the PBS solution of calcium ions. The dissolution time is determined depending on the actual dosage, such as 12 hours or 24 hours.

Optionally, the volume ratio between the PBS solution of sodium alginate and the PBS solution of calcium ions is in the range of 2:1 to 6:1.

The alginic acid solution is mixed with the aqueous solution containing calcium ions to form a hydrogel having high water content, improving the injectability so that the hydrogel can be filled in a syringe or delivery system and delivered to the cardiac injury site for treatment.

The present disclosure provides an injectable anti-heart failure hydrogel with myocardial tissue repair function, which is prepared by using the above preparation method.

The present disclosure further provides a preparation method of a calcium alginate hydrogel loaded with active substance, which includes the following steps:

Step (1) of preparing a sodium alginate system: under sterile conditions, dissolving a calculated amount of sterile, pyrogen-free sodium alginate in sterile PBS and sufficiently solubilizing the same at room temperature;

Step (2) of preparing a calcium ion system: under sterile conditions, uniformly dissolving sterile, pyrogen-free calcium salt in sterile PBS and sufficiently solubilizing the same at room temperature;

Step (3) of loading the sodium alginate system with an active substance: under sterile conditions, dissolving the active substance in the sodium alginate system prepared in step (1) and sufficiently solubilizing the same at room temperature; and

Step (4) of cross-linking of the sodium alginate system and the calcium ion system: under sterile conditions, mixing the sodium alginate system loaded with the active substance prepared in step (3) and the calcium ion system prepared in step (2) thoroughly and uniformly according to a certain ratio to form a hydrogel.

Optionally, the active material is first mixed with an aqueous solution containing calcium ions and then mixed with a sodium alginate solution.

Optionally, at least one of calcium alginate and calcium chloride is dissolved in water to obtain the aqueous solution containing calcium ions.

Optionally, the aqueous solution containing calcium ions is an aqueous solution of calcium alginate, and the concentration of calcium alginate in the aqueous solution of calcium alginate is in the range of 0.01-0.1 wt%.

Optionally, the aqueous solution containing calcium ions is an aqueous solution of calcium chloride, and the concentration of calcium chloride in the aqueous solution of calcium chloride is in the range of 1-7 wt%.

Optionally, the active substance is an aqueous solution of recombinant humanized collagen type III, and the concentration of the recombinant humanized collagen type III in the aqueous solution is in the range of 0.05-0.3 wt%.

Optionally, the content of sodium alginate in the sodium alginate solution is in the range of 2-7 wt%.

Optionally, the mass ratio between the aqueous solution containing calcium ions and the aqueous solution of recombinant humanized collagen type III when mixed is in the range of 0.1-0.9 : 0.1-0.8.

Optionally, the aqueous solution containing calcium ions is mixed with the aqueous solution of recombinant humanized collagen type III to obtain a mixed solution. The mass ratio between the mixed solution and the sodium alginate solution is in the range of 0.01-0.05 : 0.1-0.5.

The present disclosure further provides a preparation method of the injectable hydrogel for heart failure treatment with myocardial tissue repair function, including the following steps under sterile conditions:
Step 1, preparing an aqueous solution containing calcium ions, recorded as solution A; and
Preparing an aqueous solution of recombinant humanized collagen type III, recorded as solution B;
Step 2: mixing solution A and solution B uniformly to obtain solution C;
Step 3: mixing solution C with sodium alginate solution thoroughly to obtain the hydrogel.

The amino acid fragment sequence of the recombinant humanized collagen type III is: GERGAPGFRGPAGPNGIPGEKGPAGERGAP.

The composite hydrogel prepared by the above method not only improves the mechanical properties and biodegradability of humanized collagen type III, but also enhances the ability of calcium alginate to repair and regenerate tissues. Therefore, the hydrogel can promote the repair and regeneration of injured tissues while maintaining the normal function of myocardial cells and tissues, making a complete cure of heart failure possible.

The composite hydrogel prepared by the above method is less likely to be hydrolyzed by enzymes, can repair tissues, and induce normal proliferation and growth of cells and tissues.

Optionally, at least one of calcium alginate and calcium chloride is dissolved in water to obtain the aqueous solution containing calcium ions.

Optionally, the aqueous solution containing calcium ions is an aqueous solution of calcium alginate, and the concentration of calcium alginate in the aqueous solution of calcium alginate is in the range of 0.01-0.1 wt%.

Optionally, the aqueous solution containing calcium ions is an aqueous solution of calcium chloride, and the concentration of calcium chloride in the aqueous solution of calcium chloride is in the range of 1-7 wt%.

Optionally, the concentration of recombinant humanized collagen type III in the aqueous solution of recombinant humanized collagen type III is in the range of 0.05-0.3 wt%.

Optionally, the content of sodium alginate in the sodium alginate solution is in the range of 2-7 wt%.

Optionally, the mass ratio between solution A and solution B when mixed is in the range of 0.1-0.9 : 0.1-0.8.

Optionally, the mass ratio between solution A and solution B when mixed is in the range of 1-1.125 : 1.

Optionally, the mass ratio between solution C and sodium alginate solution is in the range of 0.01-0.05 : 0.1-0.5.

Optionally, the mass ratio between solution C and sodium alginate solution is in the range of 1 : 2-50.

The present disclosure further provides a preparation method of a hydrogel that can promote myocardial tissue repair and regeneration, which includes the following steps: (1) preparing medical grade CaCl₂ solution A with a concentration in the range of 0.01-0.1 wt%, and setting aside; (2) weighing 0.001-0.006 g recombinant humanized collagen type III powder to a 15 mL centrifuge tube, dropwise adding 2 g of distilled water, shaking and mixing to obtain collagen aqueous solution B; (3) measuring 0.1-0.9 g solution A and 0.1-0.8 g solution B respectively and mixing them to obtain a mixed solution C; (4) transferring the mixed solution C to a 3 mL syringe 1, and setting aside; weighing 0.1-0.7 g medical grade sodium alginate solution to a 3 mL syringe 2, (5) using a three-way valve to connect syringe 1 and syringe 2 and uniformly mixing solution A and solution B through 20-40 injection cycles to obtain the end hydrogel product of calcium alginate/recombinant humanized collagen type III; (6) storing the hydrogel product in an environment of 0-10 °C.

The hydrogel of calcium alginate/recombinant humanized collagen type III is prepared by physical mixing. The hydrogel has strong mechanical strength, is less likely to be degraded by enzymes, has a unique three-dimensional mesh microstructure, can repair injured tissue and induce tissue regeneration.

The advantage of the above method is that the composite hydrogel of calcium alginate/recombinant humanized collagen type III is prepared through simple physical mixing. The hydrogel has a three-dimensional mesh structure with a mesh aperture of 50 µm, with a storage modulus in the range of 100-7000 Pa, providing a new material for the treatment of heart failure.

The present disclosure further provides a preparation method of a hydrogel that can promote myocardial tissue repair and regeneration, which includes the following steps: (1) preparing medical grade aqueous solution of calcium alginate A in the range of 0.01-0.1 wt%, and setting aside; (2) weighing 0.001-0.006 g recombinant humanized collagen type III powder to a 15 mL centrifuge tube, dropwise adding 2 g of distilled water, shaking and mixing to obtain collagen aqueous solution B; (3) measuring 0.1-0.9 g solution A and 0.1-0.8 g solution B respectively and mixing them to obtain a mixed solution C; (4) transferring the mixed solution C to a 3 mL syringe 1, and setting aside; weighing 0.1-0.7 g medical grade sodium alginate solution to a 3 mL syringe 2, (5) using a three-way valve to connect syringe 1 and syringe 2 and uniformly mixing solution A and solution B through 20-40 injection cycles to obtain the end hydrogel product of calcium alginate/recombinant humanized collagen type III; (6) storing the hydrogel product in an environment of 0-10 °C.

The advantage of the hydrogel prepared by the present disclosure is that it can repair injured tissues, promote the proliferation and normal growth of cells and tissues, is less likely to be hydrolyzed by enzymes, has excellent mechanical strength, and can maintain repair and regeneration performance for a long time. The hydrogel makes it possible to completely cure heart failure and has important potential value in the biomedical field.

The present disclosure further provides a hydrogel for repairing cardiac injury. The hydrogel is the above injectable hydrogel for heart failure treatment with myocardial tissue repair function that acts on the cardiac injury site.

The present disclosure further provides a hydrogel for treating heart failure. The hydrogel is the injectable hydrogel for heart failure treatment with myocardial tissue repair function that acts on the cardiac diseased site.

The present disclosure further provides a hydrogel for treating myocardial infarction. The hydrogel is the injectable hydrogel for heart failure treatment with myocardial tissue repair function that acts on the myocardial infarction site.

The present disclosure further provides a use of the injectable hydrogel for heart failure treatment with myocardial tissue repair function in repairing cardiac injury.

The present disclosure further provides a use of the injectable hydrogel for heart failure treatment with myocardial tissue repair function in treating heart failure.

The present disclosure further provides a use of the injectable hydrogel for heart failure treatment with myocardial tissue repair function in treating myocardial infarction.

The present disclosure further provides a method of repairing cardiac injury, including administering the injectable hydrogel for heart failure treatment with myocardial tissue repair function to the cardiac injury site.

The present disclosure further provides a method of repairing cardiac injury, including injecting the injectable hydrogel for heart failure treatment with myocardial tissue repair function into the cardiac diseased site.

The present disclosure further provides a method of treating heart failure, including administering the injectable hydrogel for heart failure treatment with myocardial tissue repair function to the cardiac diseased site.

The present disclosure further provides a method of treating heart failure, including injecting the injectable hydrogel for heart failure treatment with myocardial tissue repair function into the myocardial infarction site.

The present disclosure further provides a method of treating myocardial infarction, including involves injecting the injectable hydrogel for heart failure treatment with myocardial tissue repair function into the myocardial infarction site.

The hydrogel loaded with an active substance according to the present disclosure has at least one of the following benefits:
1) The calcium alginate hydrogel loaded with an active substance can be quickly formed by the chelation of many carboxylic acid groups of alginic acid and multivalent cations;
2) The hydrogel has a simple preparation process and has excellent rheological properties and injectability;
3) The hydrogel can provide mechanical support, and significantly promote cell growth, which can effectively promote the formation and repair of blood vessels in the injured heart.

### BRIEF DESCRIPTION OF DRAWINGS

FIG. 1a is an image of sodium alginate PBS solution;
FIG. 1b is an image of hydrogel group 1;
FIG. 1c is an image of hydrogel group 2;
FIG. 2a is a scanning electron microscope image of hydrogel group 1 according to Example 1 of the present disclosure;
FIG. 2b is a scanning electron microscope image of hydrogel group 2 according to Example 1 of the present disclosure;
FIG. 3a is an image showing the injectability of hydrogel group 1 according to Example 1 of the present disclosure;
FIG. 3b is an image showing the injectability of hydrogel group 2 according to Example 1 of the present disclosure;
FIG. 4 is an alternating step strain sweep chart of hydrogel according to Example 1 of the present disclosure;
FIG. 5a shows the cytotoxicity result of hydrogel according to Example 1 of the present disclosure on HUVEC cells;
FIG. 5b shows the cytotoxicity result of hydrogel according to Example 1 of the present disclosure on H9C2 cells;
FIG. 6 shows cardiac ultrasound results of the hydrogel according to Example 1 of the present disclosure on the 7^{th} and 14^{th} days;
FIG. 7 shows the preparation process of the composite hydrogel of calcium alginate/recombinant humanized collagen type III according to Examples 5-9;
FIG. 8 is a rheological mechanical property chart of the composite hydrogel of calcium alginate/recombinant humanized collagen type III according to Example 5;
FIG. 9 is a rheological mechanical property chart of the composite hydrogel of calcium alginate/recombinant humanized collagen type III according to Example 6;
FIG. 10 is a rheological mechanical property chart of the composite hydrogel of calcium alginate/recombinant humanized collagen type III according to Example 7;
FIG. 11 is a rheological mechanical property chart of the composite hydrogel of calcium alginate/recombinant humanized collagen type III according to Example 8;
FIG. 12 is a rheological mechanical property chart of the composite hydrogel of calcium alginate/recombinant humanized collagen type III according to Example 9;
FIG. 13 shows an infrared spectrum of the composite hydrogel of calcium alginate/recombinant humanized collagen type III according to Example 9; and
FIG. 14 is an SEM image of the composite hydrogel of calcium alginate/recombinant humanized collagen type III according to Example 9.

### DDESCRIPTION OF EMBODIMENTS

In the following examples, reagents are chemically pure reagents unless otherwise specified.

The core amino acid fragment sequence of the recombinant humanized collagen type III used in the following examples is GERGAPGFRGPAGPNGIPGEKGPAGERGAP.

### Example 1

A preparation method of an injectable hydrogel for heart failure treatment with myocardial tissue repair function is provided, which includes the following steps.
(1) Preparing a sodium alginate system
   Under sterile conditions, 20 mg of sodium alginate was accurately weighed and dissolved in 2 mL of sterile PBS, and stirred at room temperature overnight and sufficiently solubilized;
(2) Preparing a calcium lactate system
   Under sterile conditions, 10 mg of calcium lactate was accurately weighed and dissolved in 1 mL of sterile PBS, and stirred at room temperature overnight and sufficiently solubilized;
(3) Loading a calcium alginate system with an active substance
   Under sterile conditions, 10 mg of bioactive substance of recombinant humanized collagen type III was dissolved in 2 mL of sodium alginate solution and sufficiently solubilized at room temperature;
(4) Preparing a gel from sodium alginate and calcium lactate
   350 µL of calcium lactate solution was slowly added into 2 mL of sodium alginate solution being stirred at room temperature, and immediately cross-linked to form a hydrogel.

### Example 2

A preparation method of an injectable hydrogel for heart failure treatment with myocardial tissue repair function is provided, which includes the following steps:
(1) Preparing a sodium alginate system
   Under sterile conditions, 10 mg of sodium alginate was accurately weighed and dissolved in 1 mL of sterile PBS, and stirred at room temperature overnight and sufficiently solubilized;
(2) Preparing a calcium carbonate system
   Under sterile conditions, 10 mg of calcium carbonate was accurately weighed and dissolved in 1 mL of sterile PBS, and stirred at room temperature overnight and sufficiently solubilized;
(3) Loading a calcium alginate system with an active substance
   Under sterile conditions, 5 mg of bioactive substance of recombinant humanized collagen type III was dissolved in sodium alginate solution and sufficiently solubilized at room temperature;
(4) Preparing a gel from sodium alginate and calcium carbonate
   0.5 mL of calcium carbonate solution was slowly added into 1 mL of sodium alginate solution being stirred at room temperature, and immediately cross-linked to form a hydrogel.

### Example 3

A preparation method of an injectable hydrogel for heart failure treatment with myocardial tissue repair function is provided, which includes the following steps:
(1) Preparing a sodium alginate system
   Under sterile conditions, 10 mg of sodium alginate was accurately weighed and dissolved in 1 mL of sterile PBS, and stirred at room temperature overnight and sufficiently solubilized;
(2) Preparing a calcium gluconate system
   Under sterile conditions, 10 mg of calcium gluconate was accurately weighed and dissolved in 1 mL of sterile PBS, and stirred at room temperature overnight and sufficiently solubilized;
(3) Loading a calcium alginate system with an active substance
   Under sterile conditions, 8 mg of bioactive substance of recombinant humanized collagen type III was dissolved in sodium alginate solution and sufficiently solubilized at room temperature;
(4) Preparing a gel from sodium alginate and calcium gluconate
   1 mL of calcium gluconate solution was slowly added into 1 mL of sodium alginate solution being stirred at room temperature, and immediately cross-linked to form a hydrogel.

### Example 4

A preparation method of an injectable hydrogel for heart failure treatment with myocardial tissue repair function is provided, which includes the following steps:
(1) Preparing a sodium alginate system
   Under sterile conditions, 20 mg of sodium alginate was accurately weighed and dissolved in 1 mL of sterile PBS, and stirred at room temperature overnight and sufficiently solubilized;
(2) Preparing a calcium citrate system
   Under sterile conditions, 10 mg of calcium citrate was accurately weighed and dissolved in 1 mL of sterile PBS, and stirred at room temperature overnight and sufficiently solubilized;
(3) Loading a calcium alginate system with an active substance
   Under sterile conditions, a bioactive substance of recombinant humanized collagen type III was dissolved in sodium alginate solution and sufficiently solubilized at room temperature;
(4) Preparing a gel from sodium alginate and calcium citrate
   1 mL of calcium citrate solution was slowly added into 1 mL of sodium alginate solution being stirred at room temperature, and immediately cross-linked to form a hydrogel.

### Example 5

A preparation method of a composite hydrogel of calcium alginate/recombinant humanized collagen type III in this example includes the following steps:
(1) Preparing medical grade CaCl₂ solution A with a concentration of 0.01 wt%, and setting aside;
(2) Weighing 0.004 g recombinant humanized collagen type III powder and transferring the same into a 15 mL centrifuge tube, dropwise adding 2 g distilled water, shaking and mixing to obtain collagen aqueous solution B;
(3) Measuring 0.2 g solution A and 0.1 g solutions B respectively and mixing them to obtain a mixed solution C; and
(4) Transferring the mixed solution C into a syringe, recorded as syringe 1;

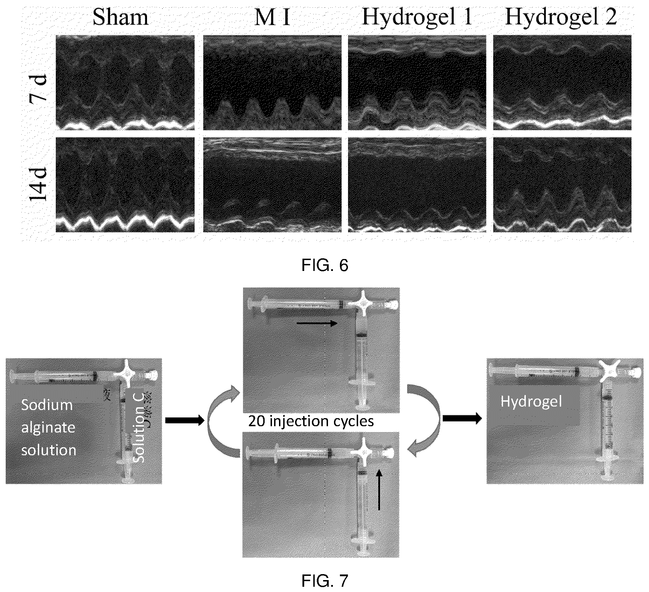

Weighing 0.4 g medical grade sodium alginate solution and transferring the same into a 3 mL syringe 2;
As shown in FIG. 7, using a three-way valve to connect syringe 1 and syringe 2, and uniformly mixing the liquids in syringe 1 and syringe 2 through 20 injection cycles to obtain the end hydrogel product of calcium alginate/recombinant humanized collagen type III; storing the hydrogel product in an environment of 0-10 °C.

The microstructure of the resulted composite hydrogel is a three-dimensional mesh structure, and the storage modulus is 161 Pa.

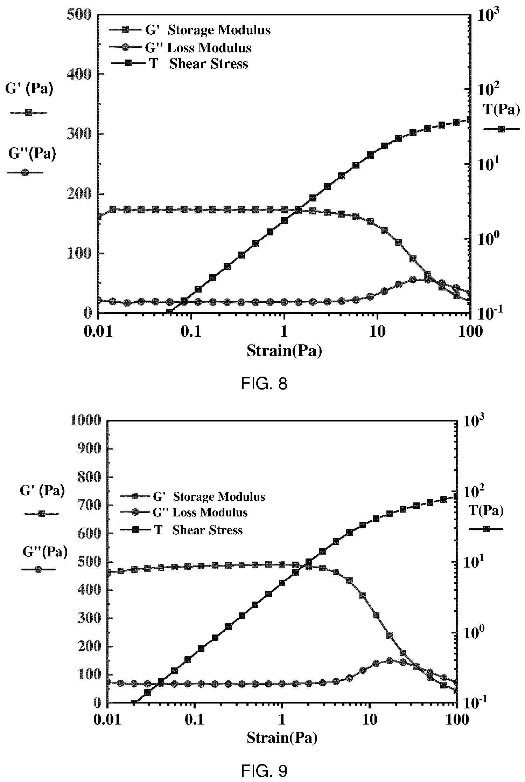

The rheological and mechanical properties of the composite hydrogel of calcium alginate/recombinant humanized collagen type III are shown in FIG. 8. When the shear stress increases to above 1 Pa, the storage modulus of the hydrogel begins to decrease and the loss modulus gradually increases. When the shear stress exceeds 62 Pa, the storage modulus of the hydrogel is less than the loss modulus, and the structure of the hydrogel is destroyed, which shows that the hydrogel has a shear-thinning property of an injectable hydrogel.

### Example 6

(1) Preparing medical grade CaCl₂ solution A with a concentration of 0.01 wt%, and setting aside;
(2) Weighing 0.004 g recombinant humanized collagen type III powder and transferring the same into a 15 mL centrifuge tube, dropwise adding 2 g distilled water, shaking and mixing to obtain collagen aqueous solution B;
(3) Measuring 0.2 g solution A and 0.3 g solutions B respectively and mixing them to obtain a mixed solution C; and
(4) Transferring the mixed solution C into a syringe, recorded as syringe 1;

Weighing 0.4 g medical grade sodium alginate solution and transferring the same into a 3 mL syringe 2;
Using a three-way valve to connect syringe 1 and syringe 2, and uniformly mixing the liquids in syringe 1 and syringe 2 through 30 injection cycles to obtain the end hydrogel product of calcium alginate/recombinant humanized collagen type III; storing the hydrogel product in an environment of 0-10 °C.

The microstructure of the resulted composite hydrogel is a three-dimensional mesh structure, and the storage modulus is 462 Pa.

The rheological and mechanical properties of the composite hydrogel of calcium alginate/recombinant humanized collagen type III are shown in FIG. 9.

### Example 7

(1) Preparing medical grade CaCl₂ solution A with a concentration of 0.01 wt%, and setting aside;
(2) Weighing 0.004 g recombinant humanized collagen type III powder and transferring the same into a 15 mL centrifuge tube, dropwise adding 2 g distilled water, shaking and mixing to obtain collagen aqueous solution B;
(3) Measuring 0.2 g solution A and 0.4 g solutions B respectively and mixing them to obtain a mixed solution C; and
(4) Transferring the mixed solution C into a syringe, recorded as syringe 1;

Weighing 0.4 g medical grade sodium alginate solution and transferring the same into a 3 mL syringe 2;
Using a three-way valve to connect syringe 1 and syringe 2, and uniformly mixing the liquids in syringe 1 and syringe 2 through 40 injection cycles to obtain the end hydrogel product of calcium alginate/recombinant humanized collagen type III; storing the hydrogel product in an environment of 0-10 °C.

The microstructure of the resulted composite hydrogel is a three-dimensional mesh structure, and the storage modulus is 648 Pa.

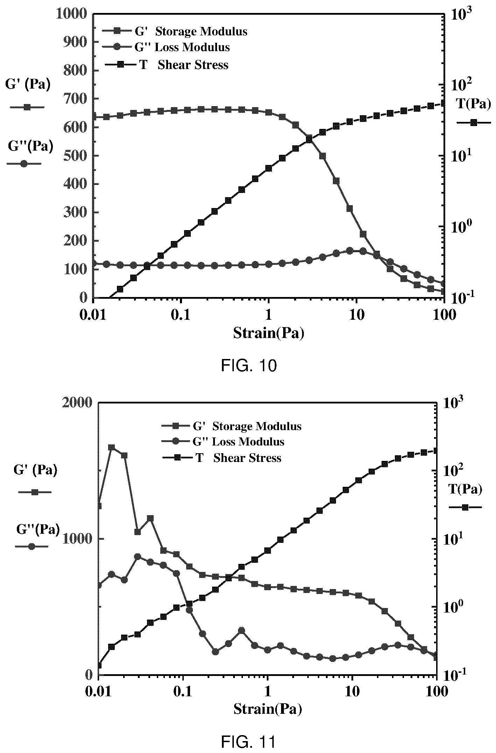

The rheological and mechanical properties of the composite hydrogel of calcium alginate/recombinant humanized collagen type III are shown in FIG. 10.

### Example 8

(1) Preparing medical grade CaCl₂ solution A with a concentration of 0.01 wt%, and setting aside;
(2) Weighing 0.004 g recombinant humanized collagen type III powder and transferring the same into a 15 mL centrifuge tube, dropwise adding 2 g distilled water, shaking and mixing to obtain collagen aqueous solution B;
(3) Measuring 0.9 g solution A and 0.4 g solutions B respectively and mixing them to obtain a mixed solution C; and
(4) Transferring the mixed solution C into a syringe, recorded as syringe 1;

Weighing 0.4 g medical grade sodium alginate solution and transferring the same into a 3 mL syringe 2;
Using a three-way valve to connect syringe 1 and syringe 2, and uniformly mixing the liquids in syringe 1 and syringe 2 through 30 injection cycles to obtain the end hydrogel product of calcium alginate/recombinant humanized collagen type III; storing the hydrogel product in an environment of 0-10 °C.

The microstructure of the resulted composite hydrogel is a three-dimensional mesh structure, and the storage modulus is 1663 Pa.

The rheological and mechanical properties of the composite hydrogel of calcium alginate/recombinant humanized collagen type III are shown in FIG. 11.

### Example 9

(1) Preparing medical grade CaCl₂ solution A with a concentration of 0.01 wt%, and setting aside;
(2) Weighing 0.004 g recombinant humanized collagen type III powder and transferring the same into a 15 mL centrifuge tube, dropwise adding 2 g distilled water, shaking and mixing to obtain collagen aqueous solution B;
(3) Measuring 0.8 g solution A and 0.1 g solutions B respectively and mixing them to obtain a mixed solution C; and
(4) Transferring the mixed solution C into a syringe, recorded as syringe 1;

Weighing 0.4 g medical grade sodium alginate solution and transferring the same into a 3 mL syringe 2;
Using a three-way valve to connect syringe 1 and syringe 2, and uniformly mixing the liquids in syringe 1 and syringe 2 through 30 injection cycles to obtain the end hydrogel product of calcium alginate/recombinant humanized collagen type III; storing the hydrogel product in an environment of 0-10 °C.

The microstructure of the resulted composite hydrogel is a three-dimensional mesh structure, and the storage modulus is 7000 Pa.

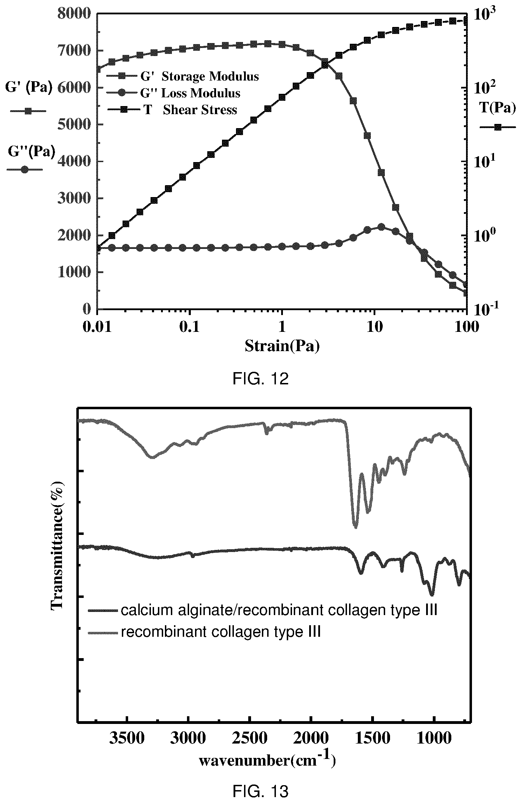

The rheological and mechanical properties of the composite hydrogel of calcium alginate/recombinant humanized collagen type III are shown in FIG. 12.

The infrared spectrum of the composite hydrogel of calcium alginate/recombinant humanized collagen type III is shown in FIG. 13, from which the amide characteristic peaks of collagen can be observed: amide I (1649-1659 cm⁻¹), amide II (1550-1561 cm⁻¹), amide III (1237-1242 cm⁻¹), amide A (3409-3426 cm⁻¹) and amide B (2932-2961 cm⁻¹).

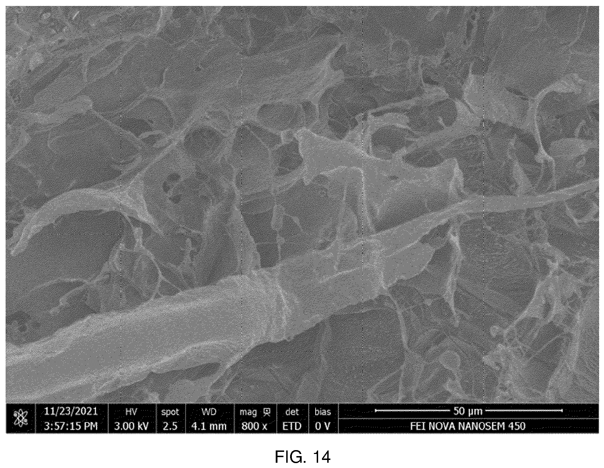

FIG. 14 is an SEM image of the composite hydrogel of calcium alginate/recombinant humanized collagen type III, which shows the hydrogel has a porous structure.

### Test example

The substance prepared in Example 1 is used as an example for test. The specific operation process and results are as follows.

Unless otherwise specified, hydrogel group 1 and group 2 are respectively the following groups: Hydrogel group 1 (Hydrogel 1): blank hydrogel; Hydrogel group 2 (Hydrogel 2): hydrogel loaded with recombinant humanized collagen type III.
1. The hydrogel prepared in step (4) was tested. Specifically, the rheological property of the hydrogel was tested with an MCR302 rheometer. A dual concentric cylinder with a gap of 4 mm was used for steady shear flow at 37 °C. Frequency sweep was based on 1% strain and oscillation frequency of 0.1-100 rad/s. The oscillation frequency of strain sweep was 1 Hz, and the strain was 0.01-1000%. In the self-repair experiment, alternating step strain sweep (large strain: 1000%, 60s, and small strain: 1%, 60s) was performed.

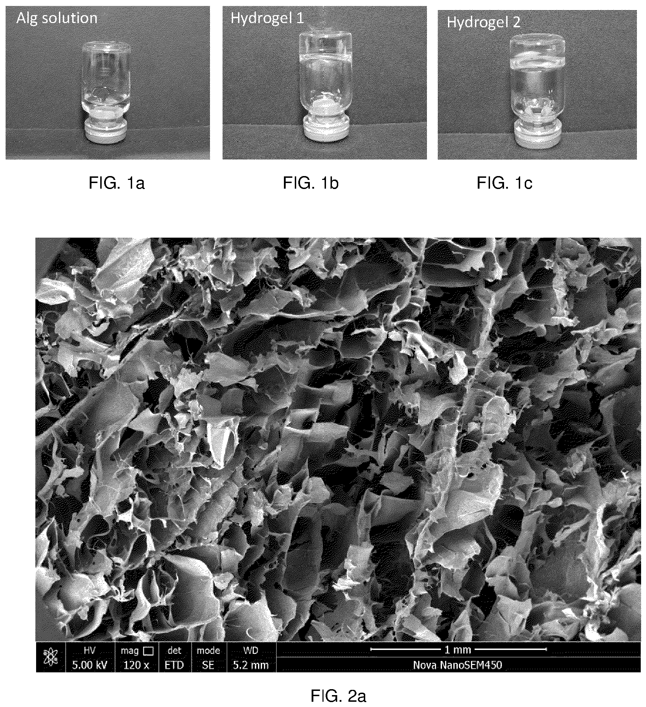

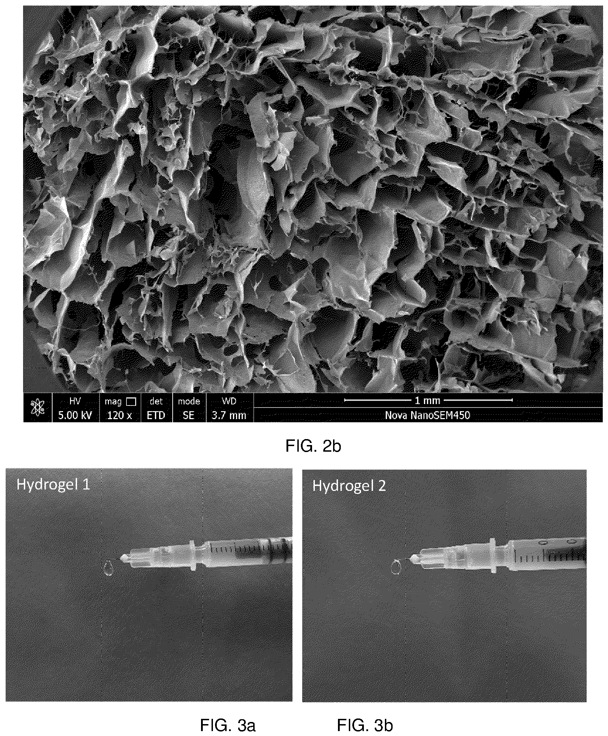

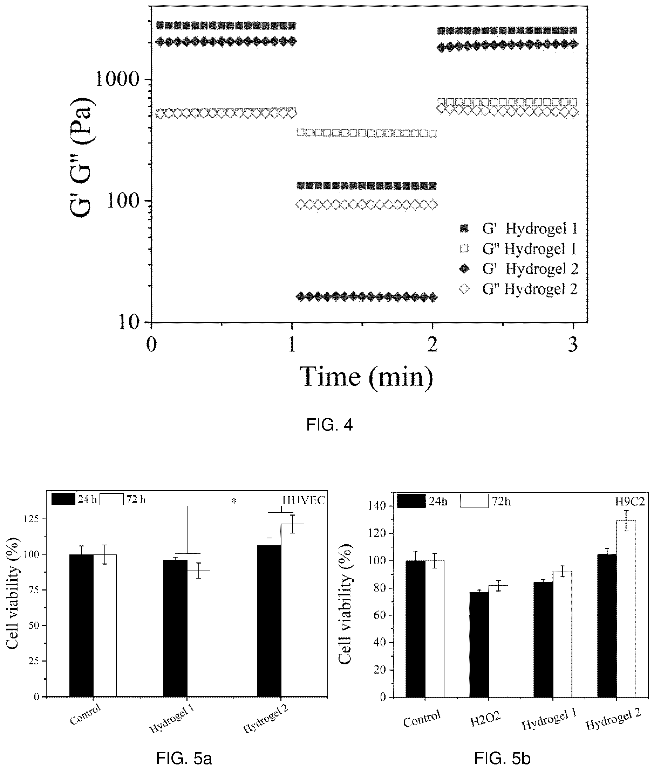

FIG. 1a is an image of sodium alginate PBS solution. FIG. 1b is an image of hydrogel group 1. FIG. 1c is an image of hydrogel group 2. FIG. 2a and FIG. 2b are scanning electron microscope images of hydrogel. FIG. 3a and FIG. 3b show the injectability of the hydrogel; the hydrogel can be injected from a 27G needle, which proves that the hydrogel is injectable. Frequency sweeping and amplitude sweeping of the hydrogel were performed, showing that the storage modulus (G') was greater than the loss modulus (G"), proving the successful preparation of the hydrogel. FIG. 4 shows the results of the alternating step strain sweep of the hydrogel, which shows that the hydrogel recovers 89.4% of its storage modulus after the structure of the hydrogel was destroyed three times, proving that the hydrogel has strong self-repair properties.

### 2. The biocompatibility of the hydrogel prepared in step (4) was tested.

Human umbilical vein endothelial cells (HUVECs) were used to evaluate the biocompatibility of the hydrogel. The ultraviolet-sterilized hydrogel was extracted in a cell culture medium (0.1 g/mL) for 48 hours to prepare a material extract. HUVECs cells were seeded in a 96-well plate at a seeding density of 8,000 cells per well. After 24 h, the cell culture medium was removed, and hydrogel extract was added to the well plate instead of different hydrogel samples. The proliferation rate and morphology of HUVECs cultured for 24h and 72h were detected by CCK-8 and FDA/PI staining respectively. HUVECs cells were stained with FDA (30 µg/mL) and PI (10 µg/mL) for 5 minutes, and observed with a fluorescence microscope. The results show that the fluorescence intensities of hydrogel group 1 and hydrogel group 2 were 1.4 and 1.8 times that of the control group respectively. After incubation for 24 h and 72 h, fresh culture medium (90 µL) and diluted CCK-8 solution (10 µL) were added to each well. After 2 h, the cell proliferation rate was calculated by measuring the absorbance at 450 nm with a microplate reader.

The results of viability of human umbilical vein endothelial cells (HUVECs)-hydrogel are shown in FIG. 5a. The results show that all hydrogel groups have no toxicity to cells at 24h and 72h. In addition, the hydrogel loaded with bioactive substance of recombinant humanized collagen type III has higher cell viability than the blank hydrogel group, indicating that recombinant humanized collagen type III can effectively promote cell proliferation and the hydrogel has good biocompatibility.

### 3. Hydrogel protects cardiomyocytes from oxidative stress damage

Rat cardiomyocytes (H9C2) and Western blotting test were used to evaluate the protein expression of hydrogel, like protecting cardiomyocytes from oxidative stress damage and reducing cardiomyocyte apoptosis. The ultraviolet-sterilized hydrogel was extracted in a cell culture medium (0.1 g/mL) for 48 hours to prepare a material extraction solution. H9C2 were seeded into a 96-well plate at a density of 8000 cells per well. After 24 h, the cell culture medium was removed, 200 µM H₂O₂ was used to pretreat H9C2 for oxidative stress damage for 1 h, and then the hydrogel extract was added to the well plate instead of different hydrogel samples. The proliferation rate and morphology of H9C2 cells cultured for 24h and 72h were detected by CCK-8 and FDA/PI staining respectively. H9C2 cells were stained with FDA (30 µg/mL) and PI (10 µg/mL) for 5 minutes, and then observed with a fluorescence microscope. The results show that compared with the control group, the cell activity of the H₂O₂ group was significantly reduced, and its fluorescence intensity was only 0.565 times that of the control group, indicating that myocardial cells suffered from oxidative stress damage and the activity was reduced. However, after adding hydrogel, the cell activity was restored to a certain extent, and the fluorescence intensities of hydrogel group 1 and hydrogel group 2 were 0.84 and 1.16 times that of the control group respectively. After incubation for 24 h and 72 h, fresh culture medium (90 µL) and diluted CCK-8 solution (10 µL) were added to each well. After 2 h, the cell proliferation rate was calculated by measuring the absorbance at 450 nm with a microplate reader.

The hydrogel-viability results are shown in FIG. 5b. The results show that all hydrogel groups have good biocompatibility for cells at 24h and 72h. In addition, the hydrogel loaded with bioactive substance of recombinant humanized collagen type III has higher cell viability than the blank hydrogel group, indicating that recombinant humanized collagen type III can protect cardiomyocytes from oxidative stress damage.

### 4. In vivo cardiac repair effect testing of hydrogel

In order to study the effect of hydrogel on cardiac repair in vivo, a rat myocardial infarction disease model was established. M-mode echocardiography detection was performed on the successfully modeled myocardial infarction rat on the 7^{th} and 14^{th} days. The results are shown in FIG. 6. The systolic and diastolic movements of the ventricular wall in the hydrogel group 2 are significantly better. On the 7^{th} and 14^{th} days, hematoxylin and eosin (H&E) staining and Masson staining were performed to evaluate the ventricular wall thickness, fibrosis degree, and collagen deposition in the MI group, hydrogel group 1, and hydrogel group 2. Histological H&E staining results in FIG. 7 show that compared with the MI control group, in hydrogel group 1 and hydrogel group 2, the ventricular walls are thicker and the fibrosis degrees are lower. Further, the ventricular wall thickness is the largest in the hydrogel group 2, which is significantly different from the hydrogel group 1 and the MI control group. The collected cardiac tissue was then stained by Masson, where blue represents collagen-rich fibrotic tissue caused by MI and red represents normal myocardial tissue. In the myocardial tissue sections on the 7^{th} and 14^{th} days, some residual gel could be observed in the hydrogel group 1 and hydrogel group 2. Compared with the MI control group, the hydrogel group 2 has less fibrotic tissue and more normal myocardial tissue. Compared with the MI control group (23.8%) and hydrogel group 1 (12.3%), the fibrosis area of hydrogel group 2 (5.9%) is significantly smaller. In summary, hydrogel can promote the regeneration of myocardial tissue and the recovery of cardiac function after myocardial infarction.

## Claims

1. An injectable hydrogel for heart failure treatment with myocardial tissue repair function, wherein the hydrogel is prepared by chelating alginic acid and multivalent cations, and the hydrogel is loaded with an active substance for repairing cardiac injury.

2. The injectable hydrogel for heart failure treatment with myocardial tissue repair function according to claim 1, wherein the active substance is recombinant humanized collagen type **III.**

3. The injectable hydrogel for heart failure treatment with myocardial tissue repair function according to claim 1 or 2, wherein the hydrogel has a storage modulus in a range of 100-7000 Pa.

4. The injectable hydrogel for heart failure treatment with myocardial tissue repair function according to claim 1 or 2, wherein the hydrogel has a three-dimensional mesh structure with a mesh aperture in a range of 30-100 µm.

5. A preparation method of the injectable hydrogel for heart failure treatment with myocardial tissue repair function according to any one of claims 1 to 4, comprising:
mixing an alginate solution with a multivalent cation solution, wherein the alginate solution and/or the multivalent cation solution contains an active substance, so as to obtain the injectable hydrogel for heart failure treatment with myocardial tissue repair function.

6. The preparation method of the injectable hydrogel for heart failure treatment with myocardial tissue repair function according to claim 5, comprising dissolving the active substance in the alginate solution and then mixing the alginate solution containing the active substance with an aqueous solution containing calcium ions.

7. The preparation method of the injectable hydrogel for heart failure treatment with myocardial tissue repair function according to claim 6, wherein the alginate solution is a PBS solution of sodium alginate, in which the sodium alginate has a concentration in a range of 0.5-5 wt%; and
the multivalent cation solution is a PBS solution of calcium ions, in which the calcium ions have a concentration in a range of 0.5-2 wt%.

8. The preparation method of the injectable hydrogel for heart failure treatment with myocardial tissue repair function according to claim 6, wherein a volume ratio between the PBS solution of sodium alginate and the PBS solution of calcium ions is in a range of 2:1 to 6:1.

9. The preparation method of the injectable hydrogel for heart failure treatment with myocardial tissue repair function according to claim 5, comprising mixing the active substance with an aqueous solution containing calcium ions and then mixing the aqueous solution containing calcium ions containing the active substance with a sodium alginate solution.

10. The preparation method of the injectable hydrogel for heart failure treatment with myocardial tissue repair function according to claim 9, wherein the aqueous solution containing calcium ions is an aqueous solution of calcium alginate, in which the calcium alginate has a concentration in a range of 0.01-0.1 wt%; or
the aqueous solution containing calcium ions is an aqueous solution of calcium chloride, in which the calcium chloride has a concentration in a range of 1-7 wt%.

11. The preparation method of the injectable hydrogel for heart failure treatment with myocardial tissue repair function according to claim 9, wherein the active substance is an aqueous solution of recombinant humanized collagen type III, in which the recombinant humanized collagen type III has a concentration in a range of 0.05-0.3 wt%.

12. The preparation method of the injectable hydrogel for heart failure treatment with myocardial tissue repair function according to claim 9, wherein sodium alginate in the sodium alginate solution has a content in a range of 2-7 wt%.

13. The preparation method of the injectable hydrogel for heart failure treatment with myocardial tissue repair function according to claim 9, wherein a mass ratio between the aqueous solution containing calcium ions and the aqueous solution of recombinant humanized collagen type III when mixed is in a range of 0.1-0.9 : 0.1-0.8.

14. The preparation method of the injectable hydrogel for heart failure treatment with myocardial tissue repair function according to claim 9, wherein a mixed solution is obtained by mixing the aqueous solution containing calcium ions and the aqueous solution of recombinant humanized collagen type III, and a mass ratio between the mixed solution and the sodium alginate solution is in a range of 0.01-0.05 : 0.1-0.5.

15. Use of the injectable hydrogel for heart failure treatment with myocardial tissue repair function according to claim 1 or 2 in repairing cardiac injury.
